# EUROPEAN PATENT SPECIFICATION

(11) **EP 2 335 597 B1**
(45) Date of publication and mention of the grant of the patent: **01.02.2017**
(21) Application number: 10015523.3
(22) Date of filing: 10.12.2010
(51) Int. Cl.: A61B 10/00

(54) **Device for facilitating semen collection**
Verfahren zur Erleichterung der Samensammlung
Dispositif facilitant la collecte de sperme

(30) Priority: 17.12.2009 AU 2009906135; 19.11.2010 AU 2010241535
(43) Date of publication of application: 22.06.2011
(73) Proprietor: Vollrath, Klaus Michael Andreas, Mindarie WA 6030 (AU)
(72) Inventor: Vollrath, Klaus Michael Andreas, Mindarie, 6030 WA (AU); Khoury, Edward Joseph, Bateman, 6150 WA (AU)
(74) Representative: Grund, Martin

(56) References cited:
- WO-A1-2008/072086
- US-A- 2 569 702
- US-A- 4 064 760
- US-A- 5 885 233

## Description

### Field of the Invention

The invention relates to medical devices. In particular it relates to devices for collecting semen samples.

### Background of the Invention

It is not uncommon for couples wishing to have a baby to experience difficulties in conceiving. Fertility clinics exist for the very purpose of assisting people in determining the cause of the problem and, if possible, taking steps toward addressing some of the hurdles.

This typically involves the collection of semen samples for further lab testing and analysis. However, one of the difficulties commonly experienced by patients in providing the semen sample is getting the sample into the container or cup.

One conventional semen receptacle is described in U.S. Patent No. 2,569,702, which is provided with a cover insert with an extending skirt that engages the outer surface of a cup, which receives a cap adapted to be fitted over the cover.

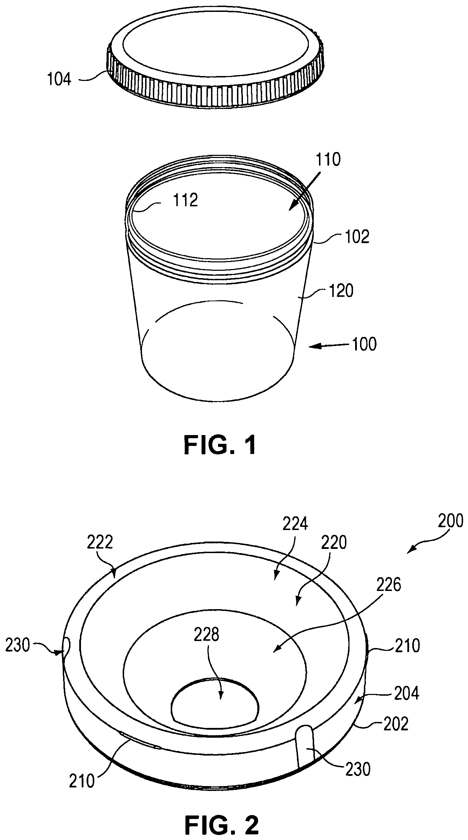

A typical cup for collecting semen samples is shown in Figure 1, and comprises a plastic cup 100 with an upper threaded portion 102 for receiving a complementarily threaded cap 104 for closing the open end 110. However, an erect penis is typically angled upwardly, thus requiring the cup 100 with its open end 110 to be essentially held upside down or at least partially inverted. This makes the task of ejaculating into the cup, while avoiding the ejaculate from flowing back out of the cup, a difficult and cumbersome process. Patients therefore often fail to provide a full ejaculate.

During the process of providing a semen sample, the patient also runs the risk of touching the inner wall 112 of the cup 100 with his penis, thereby exposing the semen sample to contaminants such as bacteria and lubricants.

The present invention seeks to address this problem by providing a new ejaculate capturing device.

### Summary of the Invention

According to the invention there is provided an insert for a semen cup according to independent claim 1. Preferred embodiments are defined in dependent claims 2 and 3.

For purposes of this application the terms "wall" and "surface" will be used in the singular to cover not only circumferentially extending walls but also multi-faceted walls with more than one wall section.

The peripheral outer surface of the insert may be defined by an outer surface of a peripheral insert wall, the insert wall having an upper end and a lower edge. The inwardly extending flange extends inwardly from the upper end of the ring shaped peripheral wall. The insert includes one or more outwardly extending tabs or a continuous outwardly extending lip engageable with an upper edge of the peripheral cup wall to avoid the insert being pushed too far into the cup.

The peripherally extending outer surface of the insert wall, or the upper portion of the inwardly extending flange preferably includes one or more breather channels for providing air flow communication between the inside of the cup and the outside when the insert is inserted into the cup.

### Brief Description of the Drawings

Figure 1 shows a three dimensional view of a prior art semen cup,
Figure 2 shows a three dimensional view of one embodiment of an insert of the invention;
Figure 3 shows a three dimensional view of another embodiment of an insert of the invention, showing the upper or outer side of the insert;
Figure 4 shows a three dimensional view of the embodiment of Figure 3, showing the lower or inner side of the insert;
Figure 5 shows a bottom view of the embodiment of Figure 3;
Figure 6 shows a sectional view along A-A of Figure 5;
Figure 7 shows a three dimensional view of a semen cup with an insert, of one embodiment of the invention and a screw cap;
Figure 8 is a sectional side view of a cup with an insert of the invention, showing the positioning of the penis in relation to the cup and insert; Figure 9 is a sectional side view of the cup and insert of Figure 8, showing the positioning of the penis positioned against the insert;
Figures 10-12 show sectional side views of a semen cup arrangement of the invention in different orientations during sampling of an ejaculate,
Figure 13 shows a sectional side view of a cup with an insert of the invention and the cup partially closed by a cap;
Figure 14 shows a sectional side view of the cup and insert of Figure 13 with the cap fully engaged with the cup;
Figure 15 shows a sectional side view making use of a modified cap;
Figure 16 shows a three dimensional view with a conventional cup, wherein an adaptor engageable with the threads of the cup is used instead of an insert;
Figure 17 shows a sectional side view making use of an insert and a modified cap;
Figure 18 shows a sectional side view of yet another embodiment of an insert and modified cap of the invention;
Figure 19 shows a three dimensional view of yet another insert, and
Figure 20 shows a three dimensional view of one embodiment of a semen cup.

### Detailed Description of the Invention

One embodiment of the invention is shown in Figure 2, which comprises an insert 200 for insertion into a prior art semen cup. It will be appreciated that the insert can be manufactured in different sizes and can be dimensioned appropriately to fit into any standard semen cup. The insert 200 has a ring-shaped configuration with a peripheral insert wall 202 having a peripherally extending outer surface 204 that is shaped and sized to fit into the open end of a semen cup (such as the open end 110 of cup 100 shown in Figure 1) to complementarily engage the inner surface 112 of the cup 100 at the open end. In the cup shown in Figure 1, the cup's peripheral wall 120 is beveled to give the cup a frusto-conical shape. Therefore, it will be appreciated that the insert 200 will be wedged in place against the inner surface of the cup's peripheral wall 120 as it is inserted deeper into the cup. However, insofar as the cup or insert are made of a flexible material such as a plastics material, the insert will typically tend to be pushed downward into the cup during use. In order to ensure that the insert 200 remains near the top of the cup, tabs 210 are included that extend outwardly from the outer surface 204 of the wall 202, at the upper end of the wall 202.

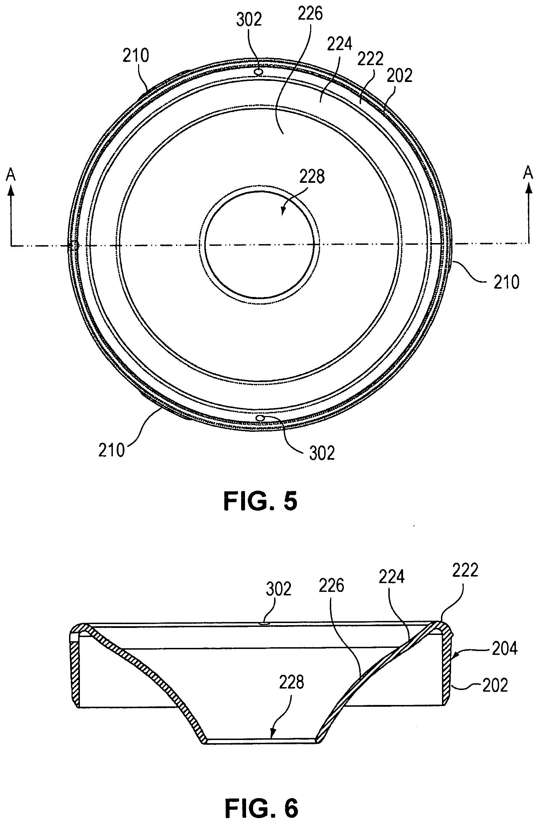

As shown in Figure 2, an inwardly extending ledge or flange 220 extends inwardly from the upper edge of the peripheral wall 202. In this embodiment the ledge or flange 220 includes a first, outermost section in the form of a slightly rounded section 222, a concave section 224, and a convex section 226 having a convex outer surface for engaging the penis. These sections are best seen in the embodiment of Figure 6, which has a similar set of sections for the flange 220. The convex section 226 defines a central hole 228 through which the ejaculate is deposited into the semen cup. Since the penis will typically engage the outer surface of the concave section 226 in a substantially air-tight seal the insert 200 in this embodiment is provided with air vents in the form of channels 230 formed in the insert's peripheral wall 202 to allow air to pass out of the cup as the ejaculate enters the cup.

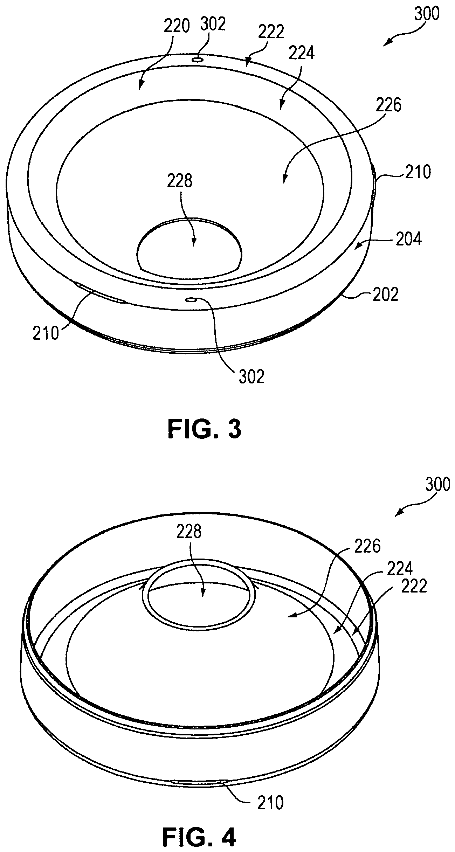

Another example is shown in Figures 3-6, which instead of air channels 230, is provided with holes 302 for venting air during use. Since this embodiment is otherwise similar to that of Figure 2, similar elements of the insert are depicted by the same reference numerals. Figure 3 shows the insert in three dimensions from the top, while Figure 4 shows the insert in three dimensions from below.

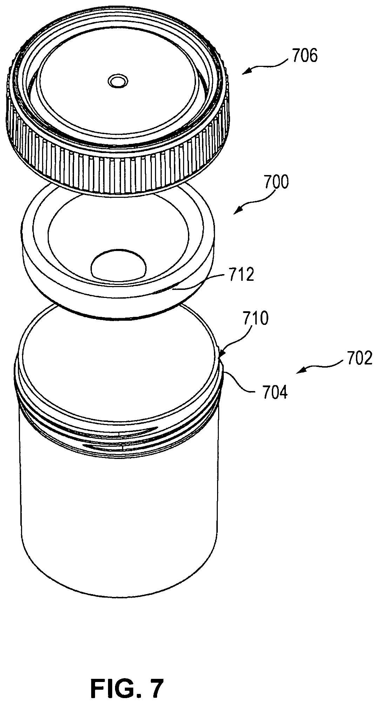

In the preferred embodiment the insert of the invention is dimensioned to fit into a standard semen cup, thereby allowing a standard closure to be used with the cup once the sample has been provided. Figure 7 shows an insert 700 of the invention in relation to a typical semen cup 702 as known in the art. The cup 702 is provided with at thread 704 at its upper outer surface to receive a complementarily threaded cap 706 as known in the art. During use, i.e., for purposes of collecting semen, the insert 700 is inserted into the cup 702 and is held in place along the upper rim 710 of the cup by tabs 712 that extend from the upper end of the peripheral wall of the insert as was discussed above.

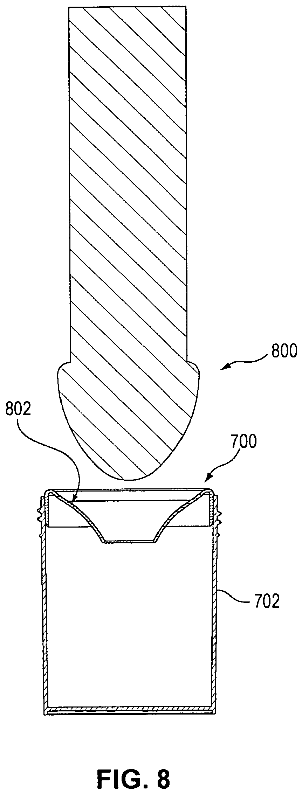

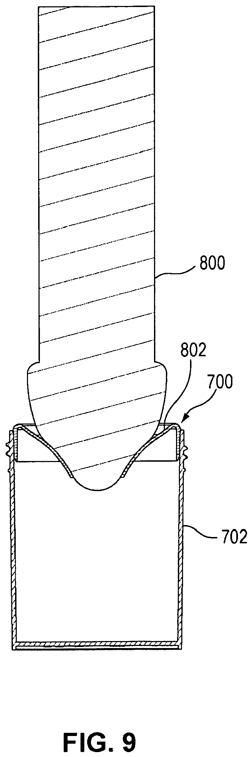

Figure 8 shows the cup 702 with the insert 700 inserted into the cup for purposes of engaging the penis 800 with the upper our outer surface of the flange 802. This is best illustrated in the sectional side view of Figure 9, which shows the penis 800 engaging mainly the convex portion of the flange 802.

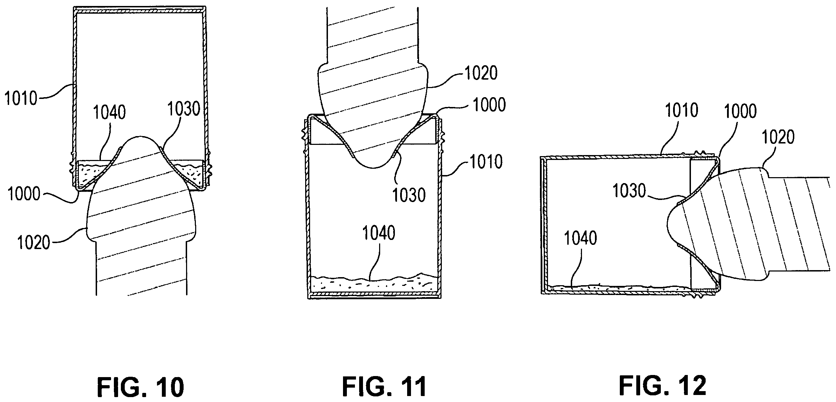

Figures 10 - 12 show an insert 1000 inserted into a standard semen cup 1010 for use in gathering a semen sample. Figure 10 shows the cup 1010 held in an inverted position with the opening of the cup and the insert 1000 toward the bottom. This positioning of the cup allows the collection of semen with the penis 1020 in a more natural, upwardly pointing orientation. The inwardly extending flange 1030 acts as a dam wall for retaining the semen 1040 inside the cup. As shown in Figure 11, the cup 1010 can be held in a conventional, substantially vertical position with the opening at the top. In this position the insert 1000 nevertheless plays an important role in that it keeps the penis 1020 and any contaminants away from the inner surface of the cup 1010. The cup may also be held in other orientations to accommodate the patient, as illustrated in Figure 12, which shows the cup in a horizontal orientation.

It will therefore be appreciated that in the orientation shown in Figures 10 and 12 the insert 1000 not only keeps contaminants away from the inside of the cup but provides the additional benefit of retaining the ejaculate in the cup since the flange 1030 acts as a barrier or ledge preventing the ejaculate from flowing out.

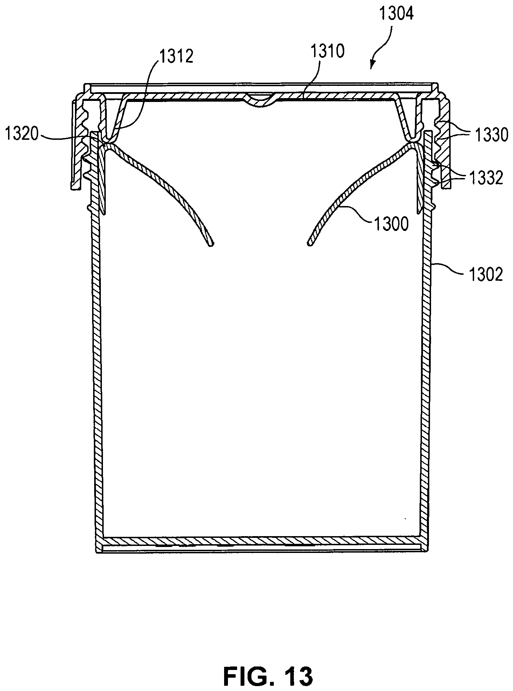

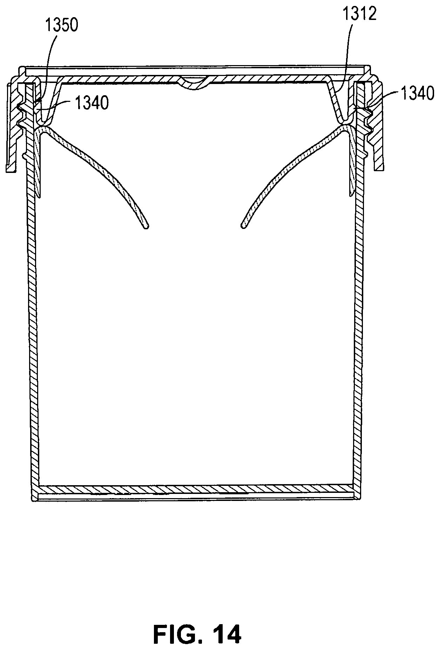

Once the semen has been deposited in the cup the insert can either be removed or remain in the cup when the cap is screwed onto the cup. As shown in Figure 13, the insert 1300 remains in the cup 1302 when the cap 1304 is placed on the cup. As shown in Figure 13, the cap 1304 has an inner wall 1310, which has a downwardly extending peripheral flange 1312 that seals with the inner surface of the side wall of the cup 1302. The sealing flange 1312 of the cap 1304 impinges upon the rounded upper portion 1320 of the inwardly extending flange of the insert 1300 before the threads 1330 of the cap have an opportunity to engage the complementary threads 1332 of the cup. Thus, as shown in Figure 13, the insert first has to be pressed further into the cap to allow the threads 1330 to catch the threads 1332, whereafter the cap 1304 can be screwed onto the cup 1302, thereby further pressing the insert 1300 downwardly into the cup, as shown in Figure 14. The peripheral flange 1312 of the cap is provided with a peripherally extending rib 1340 which acts as a seal against the inner surface 1350 of the cup wall.

It will be appreciated that the tabs 210 (Figure 2) are chosen to be small enough to allow the insert to be pushed into the cup when sufficient pressure is exerted on the insert, while acting retaining the insert at the top of the cup during use (ejaculation) by the patient.

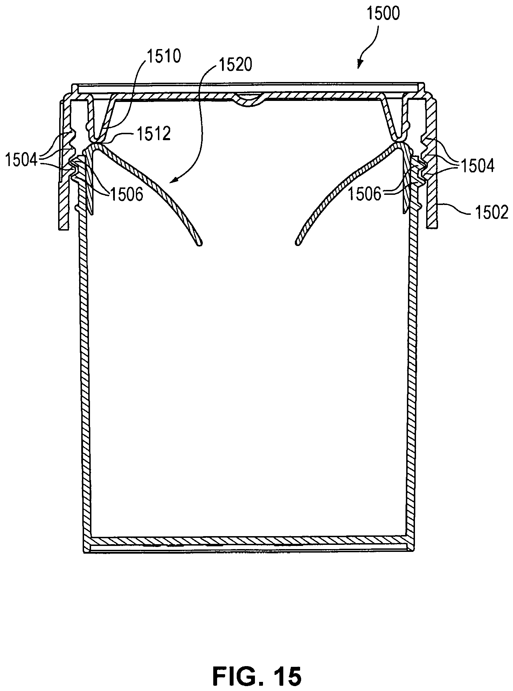

In order to avoid the two step process in closing the cup after use, namely first pressing down on the cap to push the insert down far enough to allow the threads on the cap and cup to engage one another, and then screwing the cap down, also disclosed is the use of a cap with a longer peripheral wall as shown in Figure 15, in which the peripheral wall 1502 of the cap 1500 is made longer to allow the threads 1504 on the inner surface of the peripheral wall 1502 to be positioned lower on the cap and thereby allow the threads 1504 to engage the threads 1506 of the cap before the flange 1510 on the cap impinges upon the upper rounded section 1512 of the insert 1520. When the cap is thereafter screwed downward onto the cup, the flange 1510 presses the insert 1520 downward into the cup.

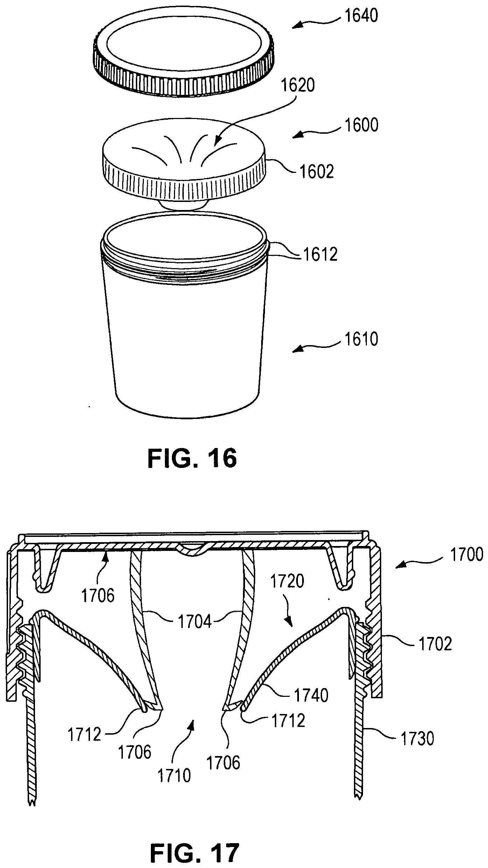

Also disclosed is when the penis-engaging adaptor is implemented not as an insert as in the above embodiments but as a separate screw-on adaptor 1600 as shown in Figure 16. Here, the peripheral wall 1602 of the adaptor 1600 is provided with an inner thread (not visible) for engaging the threads 1612 on the cup 1610. The inwardly extending flange 1620 on the adaptor 1600 is similar to the flange 220, 802 described above. This, however, has the benefit that the user can remove the adaptor 1600 after the ejaculate has been deposited in the cup, thereby allowing the regular cap 1640 to be screwed onto the cup without interference by an insert. A further benefit is that lab technicians dealing with the semen sample have easier access to the semen and are not hampered by an insert in the cup.

In Figure 17, the regular cap is replaced with a cap similar to that described with respect to Figure 15. However, the cap 1700, in addition to the longer peripheral wall 1702 is provided with four flexible fingers 1704 extending downwardly from the inner surface 1706 of the cap. The fingers 1704 are positioned so that there lower extremities are spaced further apart than the diameter of the central opening 1710 of the insert 1720. The fingers 1704 are also provided with outwardly extending hooks 1714. Thus, when the cap 1700 is screwed down on the cup 1730, the central hooks move downward, being forced inward by the slanted surface of the flange 1740. Once the hooks 1706 pass through the opening 1710 they spring back underneath the lower edge 1712 of the insert 1720. Thus when the cap 1700 is subsequently unscrewed by lab personnel the hooks 1706 engage the lower edge 1712 of the insert 1720 and extract the insert 1720 from the cup to allow the lab personnel to access the semen sample more easily. It will be appreciated that as few as one hooked finger could be provided to extract the insert. The finger can also have different configurations. For example the finger could comprise a central post with multiple hooks extending from the post, wherein the hooks are deformable or pivotable in one direction only to facilitate insertion through the central opening of the insert, but resisting deformation or pivoting in the opposite direction, thereby allowing the cap with its post and hooks to extract the insert when the cap is unscrewed.

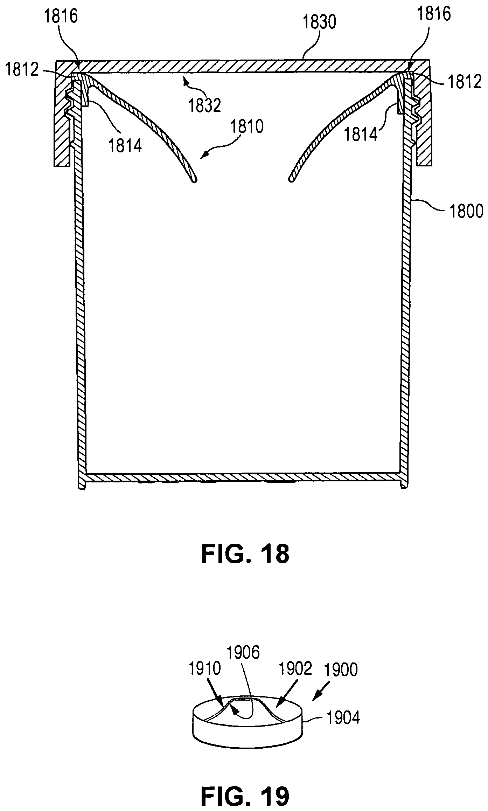

In yet another example, instead of making use of tabs e.g. tabs 210 in the Figure 2 embodiment, a retaining lip is provided that extends around the entire periphery of the insert and engages the upper rim of the cup. Such an embodiment is shown in Figure 18, which shows a conventional cup 1800 with an insert 1810 of the invention inserted into the cup 1800. As is shown in Figure 18, a lip 1812 extends from the side wall 1814 of the insert 1810 and rests on top of the rim of the cup 1800 when the insert is inserted into the cup. The cap 1830, which replaces the conventional cap described with respect to Figures 13-15, is provided without the downwardly extending flange 1312, 1510 that was seen in the embodiments of Figures 13-15. Thus the cap does not force the insert into the cup when the cap engages the cup. The cap, instead seals by engaging the inner surface 1832 of the cap 1830 with the upper surface 1816 of the lip 1812. This, arguably, does not provide as good a seal as the embodiments of Figures 13-15 but is adequate for cups that are not exposed to the pressures of vacuum powered courier pipes that are used in some laboratories.

As discussed above the peripheral flange of the insert or adaptor e.g., flange 220 included 3 sections, however it will be appreciated that the inner two sections could define a smooth outer surface, preferably having a conical, concave or convex shape. Figure 19 shows an insert 1900 in which the flange 1902 extends inwardly from the peripheral wall 1904 in one continuous section having a smooth, convex outer surface 1906. It will be appreciated that the insert 1900 shown in Figure 19 is upside-down, therefore the upper surface 1910 in this orientation is the inner surface of the flange 1902, whereas the lower surface 1906 in this orientation is the outer surface.

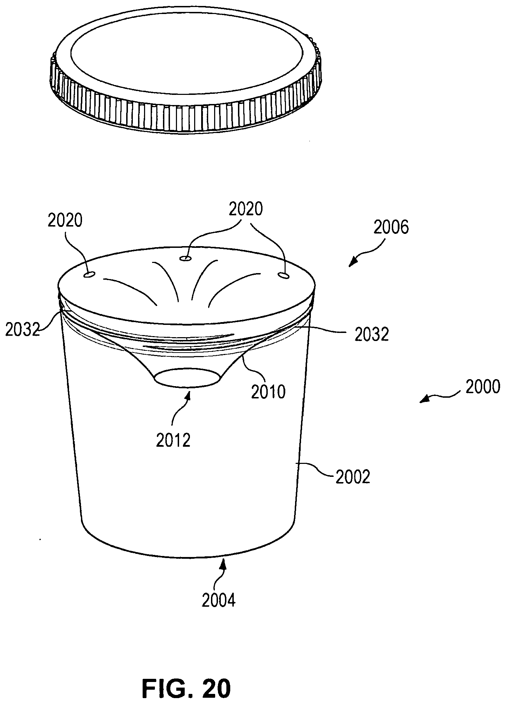

The embodiments discussed above showed the device for semen collection implemented as an insert or separate adaptor for an existing semen cup. It will be appreciated that the invention could be implemented as a new type of semen cup with built-in flange similar to the flanges 220, 1902 discussed above for the insert or adaptor. One such embodiment is shown in Figure 20. The semen cup 2000 of this embodiment is made of plastics material and is configured to be similar in size and shape to a standard prior art semen cup such as the cup shown in Figure 1. It will be appreciated that other materials such as glass can be used in other embodiments. Similar to the prior art cup 100, the cup 2000 of the present invention includes side walls 2002, a flat, closed bottom end 2004, and an upper end 2006. The cup 2000, however, distinguishes itself from the prior art cup in that it includes an inwardly extending flange 2010 with a central opening 2012. In this embodiment the flange 2010 is shown to have a slightly convex outer surface for engaging the penis during use. Air channels in the form of holes 2020 are provided in the flange 2010 to allow air to escape from the inside of the cup 2000 as semen is ejaculated into the cup. A cap 2030 is provided with internal threads (not shown) for engaging complementary threads 2032 on the outer surface of the cup. The cap 2030 is sized and shaped to correspond to existing prior art caps for easier handling, as is discussed below.

It will be appreciated that there is a benefit to sizing the inserts of the disclosed cups for use in existing semen cups or integrating the flange into cups that have a similar size to existing semen cups since laboratories that test the sample may use handling tools that are set up for existing cup sizes. For example, some laboratories use vacuum powered courier pipes to send samples from one part of the laboratory to the other, therefore in such cases the sample cups must preferably be able to withstand the pressure requirements of the vacuum powered courier pipe and also conform to the size and specifications of the courier pipe.

The size of the central opening, such as opening 228 in the embodiment of Figure 2 or the opening 2012 in the Figure 20 embodiment is also important. The central opening in the flange is preferably sized to allow only the urethral opening of the penis or a small region surrounding the urethral opening to fill the central opening of the flange. This ensures the sterility of the semen sample and keeps any contaminants on the outside. The opening in the flange also allows a sample pipette to easily pass through and reach into the corners to suction out the semen sample.

## Claims

1. An insert [200] configured for a semen cup [100], wherein the semen cup [100] includes an open end [110] and a peripheral cup wall [120] with an inner surface [112] and an outer surface, the insert comprises:
an inwardly extending flange structure [220; 1030] with a first open end defining a large inlet opening and a second open end [228] defining a smaller outlet opening, and further defining a penis-engaging surface;
wherein the inwardly extending flange structure [220; 1030] extends inwardly from the upper end of a ring-shaped peripheral wall [202], and the insert being **characterised in that** said ring-shaped peripheral insert wall [202] having a peripherally extending outer surface [204] configured to complementarily engage the inner surface [112] of the peripheral cup wall [120], and
wherein at least one of the upper portion of the inwardly extending flange structure [220; 1030] or the peripherally extending outer surface [204] of the peripheral insert wall [202] includes one or more air channels [230] or holes [302] extending therethrough for providing air flow communication between the inside of the cup [100] and the outside of the cup during use.

2. The insert [200] of claim 1, wherein the penis-engaging surface has a convex [224], concave [226] or conical portion.

3. The insert [200] of claim 1 or 2, wherein the insert [200] includes one or more outwardly extending tabs [210] or an outwardly extending peripheral lip [1812] engageable with an upper edge of the peripheral cup wall [120].

## Patentansprüche

1. Ein Einsatz [200], gestaltet für einen Samen-Becher [100], wobei der Samenbecher [100] ein offenes Ende [110] und eine randständige Becherwand [120] mit einer inneren Oberfläche [112] und einer äußeren Oberfläche umfasst, wobei der Einsatz umfasst:
eine sich nach innen erstreckende Flansch-Struktur [220; 1030] mit einem ersten offenen Ende, das eine großen Einlassöffnung definiert, und einem zweiten offenen Ende [228], das eine kleinere Auslassöffnung definiert, und außerdem eine Penisanliegende Oberfläche definierend;
wobei die sich nach innen erstreckende Flansch-Struktur [220; 1030] sich von einem oberen Ende einer ringförmigen randständigen Wand [202] nach innen erstreckt und wobei der Einsatz dadurch charakterisiert ist, dass jene ringförmige randständige Einsatzwand [202] mit einer sich randständig erstreckenden äußeren Oberfläche [204] gestaltet ist, um komplementär an der inneren Oberfläche [112] der randständigen Becherwand [120] einzurasten, und wobei mindestens eines von dem oberen Teil der sich nach innen erstreckenden Flansch-Struktur [220; 1030] oder der sich randständig erstreckenden äußeren Oberfläche [204] der randständigen Einsatzwand [202] einen oder mehrere Luftkanäle [230] oder Löcher [302] umfasst, die sich dadurch erstrecken, um während der Verwendung Luftfluss-Verbindung zwischen dem Inneren des Bechers [100] und dem Äußeren des Bechers zu bieten.

2. Der Einsatz [200] von Anspruch 1, wobei die Penis-berührende Oberfläche einen konvexen [224], konkaven [226] oder kegelförmigen Teil hat.

3. Der Einsatz [200] von Anspruch 1 oder 2, wobei der Einsatz [200] ein oder mehrere sich nach außen erstreckende Laschen [210] oder eine sich nach außen erstreckende randständige Lippe [1812] umfasst, die an einer oberen Kannte der randständigen Becherwand [120] eingerastet werden können.

## Revendications

1. Insert [200] configuré pour une coupelle à sperme [100], dans lequel la coupelle à sperme [100] inclut une extrémité ouverte [110] et une paroi périphérique de coupelle [120] avec une surface intérieure [112] et une surface extérieure, l'insert comprenant :
une structure de collerette s'étendant vers l'intérieur [220 ; 1030] avec une première extrémité ouverte définissant une grande ouverture d'entrée et une seconde extrémité ouverte [228] définissant une plus petite ouverture de sortie, et définissant en outre une surface d'engagement du pénis ;
dans lequel la structure de collerette s'étendant vers l'intérieur [220 ; 1030] s'étend vers l'intérieur depuis l'extrémité supérieure d'une paroi périphérique annulaire [202], et
l'insert étant **caractérisé en ce que** ladite paroi périphérique annulaire d'insert [202] a une surface extérieure s'étendant de façon périphérique [204] configurée pour s'engager de façon complémentaire avec la surface intérieure [112] de la paroi périphérique de coupelle [120], et dans lequel au moins l'une de la partie supérieure de la structure de collerette s'étendant vers l'intérieur [220 ; 1030] ou la surface extérieure s'étendant de façon périphérique [204] de la paroi périphérique d'insert [202] inclut un ou plusieurs canaux d'air [230] ou trous [302] s'étendant à travers celle-ci pour fournir une communication d'écoulement d'air entre l'intérieur de la coupelle [100] et l'extérieur de la coupelle durant l'utilisation.

2. Insert [200] selon la revendication 1, dans lequel la surface d'engagement du pénis présente une partie convexe [224], concave [226] ou conique.

3. Insert [200] selon la revendication 1 ou 2, dans lequel l'insert [200] inclut une ou plusieurs pattes s'étendant vers l'extérieur [210] ou un rebord périphérique s'étendant vers l'extérieur [1812] pouvant s'engager avec un bord supérieur de la paroi périphérique de coupelle [120].
